# EUROPEAN PATENT SPECIFICATION

(11) **EP 3 417 292 B1**
(45) Date of publication and mention of the grant of the patent: **24.06.2020**
(21) Application number: 17791441.3
(22) Date of filing: 10.10.2017
(51) Int. Cl.: G01N 33/564, A61K 38/22, G01N 33/68, A61K 38/33

(54) **METHODS FOR DETERMINING WHETHER A PATIENT IS LIKELY TO BENEFIT FROM TREATMENT WITH A THERAPEUTIC FORMULATION**
VERFAHREN ZUR BESTIMMUNG DER WAHRSCHEINLICHKEIT, OB EIN PATIENT VON EINER BEHANDLUNG MIT EINER THERAPEUTISCHEN FORMULIERUNG PROFITIERT
PROCÉDÉS PERMETTANT DE DÉTERMINER SI UN PATIENT EST SUSCEPTIBLE DE TIRER BÉNÉFICE D'UN TRAITEMENT COMPRENANT UNE FORMULATION THÉRAPEUTIQUE

(30) Priority: 10.10.2016 GB 201617175
(43) Date of publication of application: 26.12.2018
(73) Proprietor: Iconic Intellectual Property Limited, London W1W 8BE (GB)
(72) Inventor: MCINTOSH, Deirdre Patricia, London W1W 8BE (GB); HAQ, Syed Ebadat, London W1W 8BE (GB)
(74) Representative: Hobson, David James
(86) International application number: PCT/GB2017/053064
(87) International publication number: WO 2018/069694

(56) References cited:
- WO-A1-2014/001749
- US-A1- 2014 322 158
- N P QUILLINAN ET AL: "Treatment of diffuse systemic sclerosis with hyperimmune caprine serum (AIMSPRO): a phase II double-blind placebo-controlled trial", ANNALS OF THE RHEUMATIC DISEASES, vol. 73, no. 1, 25 September 2013 (2013-09-25), pages 56-61, XP055426969, GB ISSN: 0003-4967, DOI: 10.1136/annrheumdis-2013-203674
- NIAMH QUILLINAN ET AL: "Multiplex serum protein analysis reveals potential mechanisms and markers of response to hyperimmune caprine serum in systemic sclerosis", ARTHRITIS RESEARCH & THERAPY, vol. 19, no. 1, 7 March 2017 (2017-03-07), XP055427374, DOI: 10.1186/s13075-017-1252-x

## Description

The present disclosure relates to an assay for predicting responsive and non-responsive sub-populations of patients in a therapeutic treatment regimen.

WO 2006/021814 describes a serum composition comprising corticotropin releasing hormone (CRH), which is also known as corticotropin releasing factor (CRF) or corticoliberin. For the purposes of the present invention, references to CRH, CRF and corticoliberin are interchangeable.

WO 2006/021814 also describes the use of CRH for treating a number of disorders, in particular multiple sclerosis and inflammatory disorders such as rheumatoid arthritis; optic neuritis; motor neuron disease; autoimmune diseases; axonal or nerve damage; and cancers. Particular cancers of interest include myelomas, melanomas and lymphomas. Other disorders include cardiovascular diseases; and neural disorders, both demyelinating and non-demyelinating. Examples of particular disorders which may be treated with CRF include cerebrovascular ischemic disease; Alzheimer's disease; Huntingdon's chorea; mixed connective tissue diseases; scleroderma; anaphylaxis; septic shock; carditis and endocarditis; wound healing; contact dermatitis; occupational lung diseases; glomerulonephritis; transplant rejection; temporal arteritis; vasculitic diseases; hepatitis; and burns. Particular non-demyelinating disorders which may be treated include multiple system atrophy; epilepsy; muscular dystrophy; schizophrenia; bipolar disorder; depression; channelopathies; myasthenia gravis; pain due to malignant neoplasia; chronic fatigue syndrome; fibromyositis; irritable bowel syndrome; work related upper limb disorder; cluster headache; migraine; and chronic daily headache. Particular demyelinating disorders which may be treated include infections of the nervous system; nerve entrapment and focal injury; traumatic spinal cord injury; brachial plexopathy (idiopathic and traumatic, brachial neuritis, parsonage turner syndrome, neuralgic amyotrophy); radiculopathy; channelopathies; and tic douloureux. Particular autoimmune disorders which may be treated include lupus; psoriasis; eczema; thyroiditis; and polymyositis. Particular peripheral neuropathy of axonal and demyelinating type which may be treated include hereditary motor and sensor neuropathy of all types; Charcot-Marie-Tooth disease (CMT) types CMT1A, CMT1B, CMT2, CMT3 (Dejerine Sottas disease), CMT4 (Types A, B, C and D), X-linked Charcot-Marie-Tooth disease (CMTX); Hereditary Neuropathy with liability to pressure palsies (HNPP), also called Tomaculous neuropathy; Hereditary Motor and Sensory Neuropathy with Deafness - Lom (HMSNL); Proximal Hereditary Motor and Sensory Neuropathy/Neuronopathy (HMSNP); Hereditary Neuralgic Amyotrophy; Hereditary Sensory and Autonomic Neuropathies (HSAN1, HSAN2, HSAN3 (also called Riley-Day syndrome or familial dysautonomia), HSAN4, HSAN5); Familial Amyloid polyneuropathies (Type I, Type II, Type III, Type IV); Metachromatic Leukodystrophy; Krabbe's Disease; Fabry's Disease; Adrenoleukodystrophy; Refsum's disease (HMSN IV); Tangier Disease; Friedreich's ataxia; Spinal cerebellar ataxia (SCA) all types - SCA1, SCA2, SCA3, SCA4, SCA5, SCA6, SCA7, SCA8, SCA10, SCA11, SCA12, SCA13, SCA14, SCA16; Spinocerebellar Ataxia; Cockayne's syndrome and Giant axonal neuropathy. CRF is also identified as being useful in the treatment of chronic inflammatory demyelinating polyneuropathy (CIDP) and Guillain-Barre syndrome.

It will readily be understood that for any given treatment regime there will generally be a sub-set of patients who respond sub-optimally to the treatment and who exhibit reduced or even no improvement relative to their cohorts.

Moreover, the above list includes a variety of chronic conditions, treatment of which generally encompasses dosing a patient at regular intervals (e.g. at least twice a week) over a period of some time (i.e. several months). In view of the lengthy procedure and concomitant costs involved in treatment of these chronic conditions, it would be highly desirable to be able to identify in advance of treatment those patients who are most likely to benefit from the proposed treatment and those patients who may not be likely to benefit from the proposed treatment.

There is accordingly a need to know whether a patient is likely (or not) to benefit from treatment with a therapeutic formulation containing CRH. There is a need for a test to determine within a relatively short space of time (i.e. much less than several months into treatment) whether a patient is likely or not to benefit from treatment with a therapeutic formulation containing CRH.

US 2014/0322158 A1 describes methods of treating a subject afflicted with an autoimmune disease using predictive biomarkers of clinical response to glatiramer acetate therapy in multiple sclerosis.

WO 2014/001749 A1 describes a formulation comprising CRH and alpha-2-immunoglobulin.

Quillinan et al (2014), Ann Rheum Dis, 73:56-61 describes treatment of diffuse systemic sclerosis with hyperimmune caprine serum in a phase II double-blind placebo-controlled trial.

The present invention addresses one or more of the above needs by providing a method for determining whether a patient is likely to benefit from treatment with a therapeutic formulation, the method comprising the steps of:
(a) determining the concentration of CRH in a sample from a patient prior to administration of the therapeutic formulation;
(b) determining the concentration of CRH in a sample from a patient subsequent to administration of the therapeutic formulation; and
(c) comparing the concentration of CRH pre-administration with the concentration of CRH subsequent to administration;
wherein an increase in patient CRH concentration subsequent to administration indicates that the patient is likely to benefit from treatment with the therapeutic formulation and wherein no increase or a decrease in patient CRH concentration subsequent to administration indicates that the patient is unlikely to benefit from treatment with the therapeutic formulation; wherein the therapeutic formulation comprises CRH.

A method of treating a patient having a disorder (e.g. a disease) is described herein, the method comprising:
i. obtaining the results of a method for determining/detecting whether a patient is likely to benefit from treatment with the therapeutic formulation described herein; and
ii. administering the therapeutic formulation to said patient when it is indicated that the patient is likely to benefit from treatment with said therapeutic formulation.

Therefore, a method is described herein, the method comprising:
i. detecting the concentration of CRH in a sample from a patient prior to administration of a therapeutic formulation;
ii. detecting the concentration of CRH in a sample from a patient subsequent to administration of the therapeutic formulation;
iii. comparing the concentration of CRH pre-administration with the concentration of CRH subsequent to administration;
   wherein an increase in patient CRH concentration subsequent to administration indicates that the patient is likely to benefit from treatment with the therapeutic formulation and wherein no increase or a decrease in patient CRH concentration subsequent to administration indicates that the patient is unlikely to benefit from treatment with the therapeutic formulation; and
iv. administering the therapeutic formulation to said patient when it is indicated that the patient is likely to benefit from treatment with said therapeutic formulation.

Advantageously, pre-screening for responsiveness to treatment with a therapeutic formulation described herein allows for a more effective method of treating a patient having a disease (e.g. a disease described herein, such as multiple sclerosis or systemic sclerosis). Other benefits associated with such methods are evident to the skilled person, for example treating only responsive patients allows for more cost-efficient and/or economical prescribing of the therapeutic formulation. Alternatively or additionally, patient prognosis may be improved by way of early and/or effective treatment with a therapeutic formulation that has been shown to be effective for treating the disease in said patient.

The term "therapeutic formulation" or "formulation" as used herein means a "formulation containing CRH", i.e. a "CRH formulation". Therefore these terms are used interchangeably herein.

The methods of the invention allow for determining whether or not a patient is likely to benefit from treatment with the therapeutic formulation (i.e. whether or not a patient is likely to benefit from treatment with CRH).

The patient may be a human or non-human animal. The non-human animal may be a canine, a feline, or an equine (e.g. a dog, a cat or a horse). In one embodiment the patient is not an ungulate. In a particularly preferred embodiment the patient is a human.

The patient suitably has one or more disorder(s) (e.g. condition(s)). The disorder may involve a proliferative immune response or have an inflammatory component. In one embodiment the disorder is a neural disorder, such as a demyelinating or non-demyelinating disorder. Preferably the non-demyelinating disorder is Alzheimer's disease. Preferably the therapeutic formulation is suitable for treating said disorder.

The present inventors have found that administration of a therapeutic formulation described herein (i.e. a formulation containing CRH) can have a self-sustaining effect, in that administration of an initial amount of the formulation can lead within a matter of hours to endogenous production of CRH in the patient. It has surprisingly been found that an increase in endogenous CRH following administration of the formulation to a patient is linked with a statistically significant improvement of symptoms. More details are provided in the Examples herein. Thus the present invention provides a means by which it can be determined within a matter of hours whether a patient is likely or not to benefit from treatment with the therapeutic formulation.

The concentration of CRH in a patient may be measured by taking a sample from the patient. The sample may be a fluid sample such as blood, serum, or plasma.

The concentration of CRH subsequent to administration may be measured by taking one or more samples from the patient over a period of time. Thus, a first sample may be taken prior to administration, with one or more further samples being taken subsequent to administration. In one embodiment the sample is taken at a time when the CRH in the administered therapeutic formulation is no longer present in the patient (e.g. in the patient's blood, serum or plasma).

In one embodiment, the method can determine within 4 hours of administration whether patient CRH concentration has increased subsequent to administration and thus whether the patient is likely to benefit from treatment with the therapeutic formulation.

CRH concentration may be measured using any method known to the skilled person. In some embodiments, CRH concentration is measured by way of an antibody that binds to CRH (anti-CRH), followed by quantification of antibody binding. In some embodiments a commercial kit may be used, such as the CRF EIA Kit from Phoenix Pharmaceuticals, Inc. (Catalogue # EK-019-06).

The CRH measured in a sample from a patient subsequent to administration of a therapeutic formulation (containing CRH) is preferably the patient's endogenous CRH. For example, the CRH in the therapeutic formulation administered to the patient may be from a different source (e.g. a different genus or species) to the patient, allowing detection/distinction between the CRH in the formulation administered to the patient and the patient's CRH (production of which may be stimulated by administration of the therapeutic formulation). In such embodiments, antibodies that bind to the patient's endogenous CRH and not to the administered CRH are used. In one embodiment non-ungulate CRH is measured and the administered CRH is an ungulate CRH, or the administered therapeutic formulation (containing CRH) is derived from an ungulate. Preferably, human CRH is measured and the administered CRH is non-human CRH, or the administered therapeutic formulation (containing CRH) is derived from a non-human. More preferably, human CRH is measured and the administered CRH is an ungulate CRH, or the administered therapeutic formulation (containing CRH) is derived from an ungulate.

Corticotropin releasing hormone (CRH), also known as corticoliberin, is a 41 residue peptide originally isolated from ovine hypothalamus based on its ability to stimulate the hypothalamic-pituitary adrenal axis from cultured anterior pituitary cells. CRH is the principal neuroregulator of the basal and stress-induced secretion of ACTH, β-endorphin, and other pro-opiomelanocortin related peptides from the paraventricular nucleus of the anterior pituitary gland. In addition to its endocrine function, mediation of CRH specific responses occur via its cognate receptors (they include CRH-R1, CRH-R2α, CRH-R2β, CRH-R2γ and CRH-binding protein [CRH-BP]). CRH-R1 is a 415 amino acid protein that shows sequence homology across different species (human, mouse and rat). CRH-binding protein represents the smallest receptor at 322 amino acids, and acts as an inhibitor of free CRH. CRH-R1 and CRH-R2 are both ubiquitously expressed on the cell surface of the hypothalamus, cerebellum, cortex, amygdala, subcortex, immune cells, gut and skin. Conversely, CRH-BP is found predominantly in the liver, placenta and brain. Importantly there appears to be no significant overlap in distribution of the said receptors. This likely reflects differing functional roles. An example of this is seen during pregnancy where elevation in peripheral CRH is regulated by an elevation in secreted levels of CRH binding protein. The overall effect of this is to prevent an elevation in peripheral circulating levels of glucocorticoids during pregnancy. Several forms of CRH have been identified in nature, they include a high molecular weight form 194 amino acids *Mw* ∼ 30,000, a *Mw* ∼18,000, *Mw* ∼7,500 and the 41 amino acid residue. All three forms are biologically active and able to stimulate ACTH release.

The nucleotide and amino acid sequences of human CRH are described in GenBank (see accession numbers BC011031 and AAH11031.1, respectively).

The therapeutic formulation may be derived from any appropriate source. In one embodiment the formulation is from a non-human source. In one embodiment the CRH is an ungulate CRH. In one embodiment the therapeutic formulation is derived from an ungulate. The ungulate may be one or more selected from the following families: Bovidae, Equidae, Tapiridae, Rhinocerotidae, Camelidae, Tayassuidae, Suidae, Hippopotamidae, Tragulidae, Antilocapridae, Giraffidae, Cervidae, and Moschidae. Suitably, the ungulate may be one or more selected from a goat, a horse, a zebra, a donkey, cattle, bison, a pig, a moose, an elk, a deer, a llama, a camel, an alpaca, an antelope or a gazelle. In one embodiment the ungulate is not an ovine. Preferably the ungulate is a goat.

Therefore, a therapeutic formulation may be administered to a non-ungulate patient, wherein the CRH (comprised within the therapeutic formulation) or the therapeutic formulation, is derived from an ungulate. The concentration of non-ungulate CRH may be determined prior to and subsequent to administration of the therapeutic formulation.

The therapeutic formulation may be a hyperimmune serum, e.g. a hyperimmune serum obtainable from an ungulate.

The term "obtainable" as used herein encompasses the term "obtained". In one embodiment the term obtainable means obtained.

In one embodiment a therapeutic formulation described herein comprises CRH and alpha-2 macroglobulin. In some embodiments the CRH and alpha-2 macroglobulin exist in a complex (e.g. a stabilised complex).

Alpha-2-macroglobulin, also known as a2M, is a large plasma protein found in blood. It is produced by the liver and is the largest major non-immunoglobulin protein in plasma. A2M is synthesized primarily by the liver and is also produced locally by macrophages fibroblasts and adrenocortical cells. Alpha-2-macroglobulin acts as an anti-protease and is able to inactivate an enormous variety of proteinases. It also functions as a carrier protein binding to numerous growth factors and cytokines. Examples include transferrin (where a2M regulates binding to the surface receptor), it binds defensin and myelin basic protein, binds several important cytokines, including basic fibroblast growth factor (bFGF), platelet derived growth factor (PDGF), nerve growth factor (NGF), interleukin-1β (IL-1β) and interleukin-6 (IL-6), transforming growth factor (TGF-1β), and insulin, and modifies their biological activity. Human a2M is composed of four identical subunits bound together by -S-S- bonds. The principal mechanism by which a2M inhibits proteases is through steric hindrance. The mechanism involves protease cleavage of the thiol 35 amino acid bait region, a segment of the molecule, which is particularly susceptible to proteolytic cleavage, which initiates conformational change such that a2M collapses about the protease thus resulting in its inhibition. In the resulting a2M-protease complex, the active site of the protease is sterically shielded, thus substantially decreasing access to protein substrates including those that are bound to active a2M. Decreases in a2M have been associated with a variety of diseases, for example a common variant (29.5%) polymorphism of a2M leads to an increased risk of Alzheimer's disease.

A therapeutic formulation may further comprise CRH binding protein (CRH-BP).

In some embodiments the therapeutic formulation comprises a pro-opiomelanocortin (POMC) peptide. Following administration, *in vivo* activation of POMC may occur by the direct actions of prohormone convertase 1 (PC1), prohormone convertase 2 (PC2), carboxypeptidase E (CPE), peptidyl alpha-amidating monooxygenase (PAM), N-acetyltransferase (N-AT) and prolylcarboxypeptidase (PRCP) acting in a tissue specific manner. All said POMC cleavage sites appear to be acted upon by proteases in the hypothalamus, placenta, epithelium and leukocytes.

Human POMC peptide is described in detail in entry 176830 of OMIM (online mendelian inheritance in man, accessible through http://www.ncbi. nlm.nih.qovA). The nucleotide and amino acid sequence of human POMC is also known, and have GenBank accession numbers BC065832 and AAH65832.1, respectively. Human POMC gives rise to a glycosylated protein precursor having a molecular weight of 31 kDa.

The term "POMC peptide" as used herein refers to any peptide having a corresponding sequence, structure, or function. It will be apparent to the skilled person that the canonical nucleotide and/or amino acid sequences given for human POMC in the GenBank entry referenced above may be varied to a certain degree without affecting the structure or function of the peptide. In particular, allelic variants and functional mutants are included within this definition. Mutants may include conservative amino acid substitutions. A "POMC peptide" as used herein refers to any peptide acting as a precursor to at least one form of MSH, ACTH, at least one form of lipotrophin (LPH), β endorphin, met-enkephalin and leu-enkephalin; and preferably all of α, β, and γ MSH; ACTH; β and γ LPH; and β endorphin, met-enkephalin and leu-enkephalin.

In one embodiment the therapeutic formulation comprises CRH, alpha-2 macroglobulin, and a POMC peptide. Preferably the therapeutic formulation comprises CRH, alpha-2 macroglobulin, a POMC peptide, and CRH-BP.

The CRH, alpha-2 macroglobulin, POMC, and/or CRH-BP are preferably from a non-human. In one embodiment CRH, alpha-2 macroglobulin, POMC, and CRH-BP are ungulate (more preferably goat) CRH, alpha-2 macroglobulin, POMC, and CRH-BP.

In one embodiment the therapeutic formulation is derived from hyperimmune goat serum by the basic methodology as described in WO 2006/021814.

In more detail, a goat is immunised with HIV-3B viral lysate raised in H9 cells. The use of HIV-3B viral lysate as an immunogen is not believed to be essential for the production of active serum; it is believed that a medium which has been used for growth of a viral culture, or which is suitable for such growth, may also produce a suitable response when used as an immunogen. The supernate of a cell culture growth medium such as PBMC or the cancer immortal cell line as used to grow HIV-3B are given as an example. The HIV or other virus does not need to be present to produce an effective immunogen to create the composition. Other suitable immunogens are recited on pages 12 and 13 of WO 03/064472.

Approximately 400 cc of blood is then taken from the goat under sterile technique. The animal may typically be re-bled in 10 to 14 days, once the volume of blood is replenished. A pre-bleeding regime may be useful to stimulate production of the active components of the therapeutic formulation. The blood is then centrifuged to separate the serum, and the serum filtered to remove large clots and particulate matter. The serum is then treated with supersaturated ammonium sulphate (47% solution at 4 degrees C) to precipitate antibodies and other material. The resulting solution is centrifuged in a Beckman J6M/E centrifuge at 3500 rpm for 45 minutes, after which the supernatant fluid is removed. The precipitated immunoglobulin and other solid material are resuspended in PBS buffer (phosphate buffered saline) sufficient to redissolve the precipitate.

The solution is then subjected to diafiltration against a PBS buffer with a molecular weight cut-off of 10,000 Daltons at 4 degrees C. After diafiltration, the product is filtered through a 0.2 micron filter into a sterile container and adjusted to a protein concentration of 4 mg/ml. The solution is put into vials to give single doses of 1 ml, and stored at minus 22 degrees C prior to use.

Alternatively, a variation on this process may be employed, which includes one or more additional steps selected from (i) a nanofiltration step; (ii) avoidance of multiple freeze-thaw steps and/ or minimising ambient temperature exposure; and/ or (iii) a mixing/ agitation step. Further details of the variant manufacturing process may be found in WO 2014/001749. In one embodiment a therapeutic formulation obtainable using this process may comprise alpha-2 macroglobulin at a concentration of between 50,000 and 150,000 picograms per millilitre, for example between 100,000 and 150,000 picograms per millilitre, or between 110,000 and 130,000 picograms per millilitre. In one embodiment a therapeutic formulation obtainable using this process may comprise CRH at a concentration of between 80 picograms per millilitre (pg/ml) and 120 pg/ml, for example between 90 and 110 pg/ml. Alpha-2 macroglobulin may be present in a therapeutic formulation obtainable by this process at a concentration of between 100,000 and 150,000 picograms per millilitre, and the CRH may be present at a concentration of between 80 picograms per millilitre (pg/ml) and 120 pg/ml, for example between 90 and 110 pg/ml. The alpha-2 macroglobulin may be present in the therapeutic formulation at a concentration of between 110,000 and 130,000 picograms per millilitre, and the CRH may be present at a concentration of between 80 picograms per millilitre (pg/ml) and 120 pg/ml, for example between 90 and 110 pg/ml. A therapeutic formulation obtainable using this process may comprise CRH-BP at a concentration of between 30 and 60 picograms per millilitre (pg/ml), for example between 40 and 50 pg/ml. A therapeutic formulation obtainable using this process may comprise POMC at a concentration of between 140 picomoles per litre (pmol/l) and 200 pmol/l. In one embodiment a therapeutic formulation obtainable using this process may comprise between 110,000 and 130,000 picograms per millilitre (pg/ml) of alpha-2 macroglobulin, between 60 pg/ml and 120 pg/ml of CRH, and optionally between 140 pmol/l and 200 pmol/l of POMC.

Another variation on the method of WO 2006/021814 includes specific retention of molecules having a size greater than 0.2 microns (e.g. greater than 200 kDa). Such a method is described in WO 2015/097460. Retention of molecules having a size greater than 0.2 microns may be achieved by excluding a filtration step employing a filter pore size of 0.2 microns or less. A therapeutic formulation obtainable by this method may comprise CRH at a concentration of more than 120 pg/ml or more than 130 pg/ml. In some embodiments CRH may be at a concentration of more than 140 pg/ml or 150 pg/ml, for example more than 160 pg/ml, or more than 170 pg/ml, preferably more than 170 pg/ml, or more than 180 pg/ml, or more than 200 pg/ml. A therapeutic formulation obtainable by this method may comprise alpha-2 macroglobulin present at a concentration of more than 150,000 picograms per ml or more than 160,000 picograms per ml. In some embodiments alpha-2 macroglobulin is present in a therapeutic formulation obtainable by this method at a concentration of more than 170,000 picograms per ml, or more than 180,000 picograms per ml, for example more than 190,000 picograms per ml, or more than 200,000 picograms per ml, preferably more than 210,000 picograms per ml, or more than 220,000 picograms per ml, or more than 230,000 picograms per ml, or more than 250,000 picograms per ml. In some embodiments alpha-2 macroglobulin is present in a therapeutic formulation obtainable by this method at a concentration of more than 150,000 picograms per millilitre, and the CRH may be present at a concentration of more than 80 pg/ml. For example, the alpha-2 macroglobulin may be present at a concentration of more than 150,000 pg/ml (or more than 1 micro molar), and the CRH may be present at a concentration of more than 100 pg/ml (such as more than 120 pg/ml). A therapeutic formulation obtainable by this method may further comprise CRH binding protein (CRH-BP). The CRH-BP may be present a concentration of more than 40 or more than 50 pg/ml. The CRH-BP may be present at a concentration of more than 60 pg/ml or more than 70 pg/ml, for example more than 80 pg/ml, or more than 90 pg/ml, preferably more than 100 pg/ml, or more than 120 pg/ml, or more than 150 pg/ml. A typical upper range for CRH-BP may be in the region of 300 pg/ml. POMC may be present in a therapeutic formulation obtainable by this method at a concentration of 100 picomoles per litre (pmol/l) to 200 pmol/l.

Although the above-described methods are related to production of a hyperimmune goat serum, the person skilled in the art will appreciate that the methodology could be employed to produce a hyperimmune serum from any source, preferably from any ungulate.

The therapeutic formulation may be administered to the patient by an appropriate route (usually subcutaneously). Preferably, 1 ml of a therapeutic formulation (e.g. derived from a hyperimmune serum as described above) is administered to the patient (e.g. subcutaneously).

In one embodiment a patient is determined to be likely to benefit from treatment with a therapeutic formulation (containing CRH) when the concentration of CRH in a sample from a patient subsequent to administration of said therapeutic formulation is greater than the "baseline CRH concentration". In contrast, in one embodiment a patient is determined to be unlikely to benefit from treatment with a therapeutic formulation (containing CRH) when the concentration of CRH in a sample from a patient subsequent to administration of said therapeutic formulation is equal to or less than the "baseline CRH concentration".

The term "baseline CRH concentration" as used herein takes into account the patient's systemic CRH concentration (present in a sample described herein) as well as the increase in systemic CRH concentration owing to the CRH comprised in the administered therapeutic formulation. The "baseline CRH concentration" is thus the sum of these two concentrations. Therefore, the methods of the invention preferably allow for the measurement of an increase in CRH concentration stimulated by the administered formulation.

The increase in systemic CRH concentration owing to the CRH comprised in the administered formulation can be calculated by the skilled person based on the concentration of CRH administered to the patient. For example, where 1 ml of a formulation manufactured in accordance with WO 2014/001749 is administered to a patient, the CRH administered may be between 80 pg and 120 pg. If the sample measured from the patient is a blood sample, the average concentration increase can be calculated based on the patient's estimated blood volume and the known amount of CRH administered.

This invention will accordingly find use in a clinical trials setting.

In addition to CRH, the present inventors have found that other molecules may also act as indicators ("biomarkers") together with CRH to provide a more robust determination. Accordingly, the above-described method may additionally include measuring the concentration in a sample from the patient of one or more biomarkers selected from PIIINP and IL-17; and comparing the concentration of these biomarkers pre-administration with the concentration thereof subsequent to administration, wherein an increase in said concentration subsequent to administration indicates that the patient is likely to benefit from treatment with a therapeutic formulation described herein, and wherein no increase or a decrease in said concentration subsequent to administration indicates that the patient is unlikely to benefit from treatment with a therapeutic formulation described herein.

In one embodiment the PIIINP and/or IL-17 measured is from a different source (e.g. a different genus or species) to the administered CRH comprised in the therapeutic formulation or from which the administered therapeutic formulation is derived. In one embodiment non-ungulate PIIINP and/or IL-17 is measured and the administered CRH comprised in the therapeutic formulation is an ungulate CRH, or the administered therapeutic formulation is derived from an ungulate. Preferably, human PIIINP and/or IL-17 is measured and the administered CRH comprised in the therapeutic formulation is non-human CRH, or the administered therapeutic formulation is derived from a non-human. More preferably, human PIIINP and/or IL-17 is measured and the administered CRH comprised in the therapeutic formulation is an ungulate CRH, or the administered therapeutic formulation is derived from an ungulate.

It has been found that a test which measures the concentration of CRH together with PIIINP and/or IL-17 provides good predictive results of whether a patient is likely to respond to treatment with the therapeutic formulation. In particular, measuring the concentration of CRH and IL-17 provides good predictive results in patients suffering from multiple sclerosis. Measuring the concentration of CRH and PIINP provides good predictive results in patients suffering from systemic sclerosis.

However, good predictive results can also be obtained where only the concentration of CRH is measured.

The patient may have multiple sclerosis (MS), which may be secondary progressive multiple sclerosis (SPMS).

Additionally or alternatively the patient may have systemic sclerosis (SSc), which may be established late stage diffuse systemic sclerosis (ELSDSS).

Additionally or alternatively the patient may have one or more of the disorders selected from the following list: inflammatory disorders such as rheumatoid arthritis; optic neuritis; motor neuron disease; autoimmune diseases; axonal or nerve damage; and cancers (including myelomas, melanomas and lymphomas); cardiovascular diseases; neural disorders, both demyelinating and non-demyelinating; cerebrovascular ischemic disease; Alzheimer's disease; Parkinson's disease; Huntingdon's chorea; mixed connective tissue diseases; scleroderma; anaphylaxis; septic shock; carditis and endocarditis; wound healing; contact dermatitis; occupational lung diseases; glomerulonephritis; transplant rejection; temporal arteritis; vasculitic diseases; hepatitis (in particular hepatitis C); burns; multiple system atrophy; epilepsy; muscular dystrophy; schizophrenia; bipolar disorder; depression; channelopathies; myasthenia gravis; pain due to malignant neoplasia; chronic fatigue syndrome; fibromyositis; irritable bowel syndrome; work related upper limb disorder; cluster headache; migraine; chronic daily headache; infections of the nervous system; nerve entrapment and focal injury; traumatic spinal cord injury; brachial plexopathy (idiopathic and traumatic, brachial neuritis, parsonage turner syndrome, neuralgic amyotrophy); radiculopathy; channelopathies; tic douloureux; lupus; psoriasis; eczema; thyroiditis; polymysotis; hereditary motor and sensor neuropathy of all types; Charcot-Marie-Tooth disease (CMT) types CMT1A, CMT1B, CMT2, CMT3 (Dejerine Sottas disease), CMT4 (Types A, B, C and D), X-linked Charcot-Marie-Tooth disease (CMTX); Hereditary Neuropathy with liability to pressure palsies (HNPP), also called Tomaculous neuropathy; Hereditary Motor and Sensory Neuropathy with Deafness - Lom (HMSNL); Proximal Hereditary Motor and Sensory Neuropathy/Neuronopathy (HMSNP); Hereditary Neuralgic Amyotrophy; Hereditary Sensory and Autonomic Neuropathies (HSAN1, HSAN2, HSAN3 (also called Riley-Day syndrome or familial dysautonomia), HSAN4, HSAN5); Familial Amyloid polyneuropathies (Type I, Type II, Type III, Type IV); Metachromatic Leukodystrophy; Krabbe's Disease; Fabry's Disease; Adrenoleukodystrophy; Refsum's disease (HMSN IV); Tangier Disease; Friedreich's ataxia; Spinal cerebellar ataxia (SCA) all types - SCA1, SCA2, SCA3, SCA4, SCA5, SCA6, SCA7, SCA8, SCA10, SCA11, SCA12, SCA13, SCA14, SCA16; Spinocerebellar Ataxia; Cockayne's syndrome; Giant axonal neuropathy; chronic inflammatory demyelinating polyneuropathy (CIDP); and Guillain-Barre syndrome.

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide the skilled person with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. The headings provided herein are not limitations of the various aspects or embodiments of this disclosure.

Other definitions of terms may appear throughout the specification. Before the exemplary embodiments are described in more detail, it is to be understood that this disclosure is not limited to particular embodiments described, and as such may vary. It is also to be understood that the terminology used herein is for the purpose of describing particular embodiments only, and is not intended to be limiting, since the scope of the present disclosure will be defined only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "a therapeutic formulation" includes a plurality of such candidate agents and reference to "the therapeutic formulation" includes reference to one or more formulations and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

### BRIEF DESCRIPTION OF THE DRAWINGS

The present invention will now be described by way of example only with reference to the accompanying drawings, in which:
**Figure 1** shows the difference in mean CRH concentration within a patient group both before ("CRH pre") and 4 hours after ("CRH post") administration of a CRH formulation;
**Figure 2** shows the difference in mean scores of the 9-Hole Peg Test ("9-HPT") within a patient group having multiple sclerosis both before ("9 hole Peg pre") and 4 hours after ("9 hole Peg post") administration of the CRH formulation;
**Figure 3** shows the difference in mean scores of the 9-HPT within a second patient group having multiple sclerosis both before ("9-HPT pre_placebo") and 4 hours after ("9-HPT post_placebo") administration of a placebo;
**Figure 4** shows the difference in mean scores of the Multiple Sclerosis Functional Composite ("MSFC") within the patient group having multiple sclerosis both before ("MSFC pre") and 4 hours after ("MSFC post") administration of the CRH formulation;
**Figure 5** shows the difference in mean scores of the MSFC within the second patient group having multiple sclerosis both before ("MSFC pre placebo") and 4 hours after ("MSFC post placebo") administration of the placebo;
**Figure 6** shows the difference in Mean Rodnan Skin Score ("MRSS") within a patient group having systemic sclerosis both before ("MRSS CRH pre") and 4 hours after ("MRSS CRH post") administration of the CRH formulation;
**Figure 7** shows the difference in MRSS within a patient group having systemic sclerosis both before ("MRSS placebo pre") and 4 hours after ("MRSS placebo post") administration of the placebo;
**Figure 8** shows the relationship between the change in CRH concentration 4 hours after administration of the CRH formulation (x-axis) and the difference in MRSS clinical score (y-axis) within a patient group having systemic sclerosis;
**Figure 9** shows the relationship between the change in IL-17 concentration 4 hours after administration of the CRH formulation (x-axis) and the difference in MSFC clinical score (y-axis) within a patient group having multiple sclerosis; and
**Figure 10** shows the relationship between the change in PIIINP concentration 4 hours after administration of the CRH formulation (x-axis) and the difference in MRSS clinical score (y-axis) within a patient group having systemic sclerosis.

### EXAMPLES

### Materials & Methods

The tests (MSFC and 9-HPT) carried out herein on patients diagnosed with multiple sclerosis are described in detail in Fischer et al, Mult. Scler. (1999), 5: 244; and in Fischer et al (2001), MSFC Administration and Scoring Manual, National Multiple Sclerosis Society.

The MRSS test carried out herein on patients diagnosed with systemic sclerosis is a widely-used 17-site skin assessment described by Furst et al, J. Rheumatol. (1998), Jan; 25(1): 84-88.

CRH concentration was measured throughout using the CRF EIA Kit from Phoenix Pharmaceuticals, Inc. (Catalog # EK-019-06). The complete protocol is provided with the kit and is described in detail in Kodomari et al, Neurochem Int. (2009), Mar-Apr;54(3-4):222-8; Wang et al, PLoS One (2008), Oct 3;3(10):e3320; Ng et al, Neonatology (2008), 94(3):170-5; Moeser et al, Am J Physiol Gastrointest Liver Physiol. (2007), Aug;293(2):G413-21.

### EXAMPLE 1

### Patient CRH concentration increases following administration of the CRH formulation

A group of 35 patients was tested to establish their CRH concentration and subsequently administered 1 ml of the above-described CRH formulation by subcutaneous injection. Four hours after administration of the CRH formulation, the patients' CRH concentration was tested again.

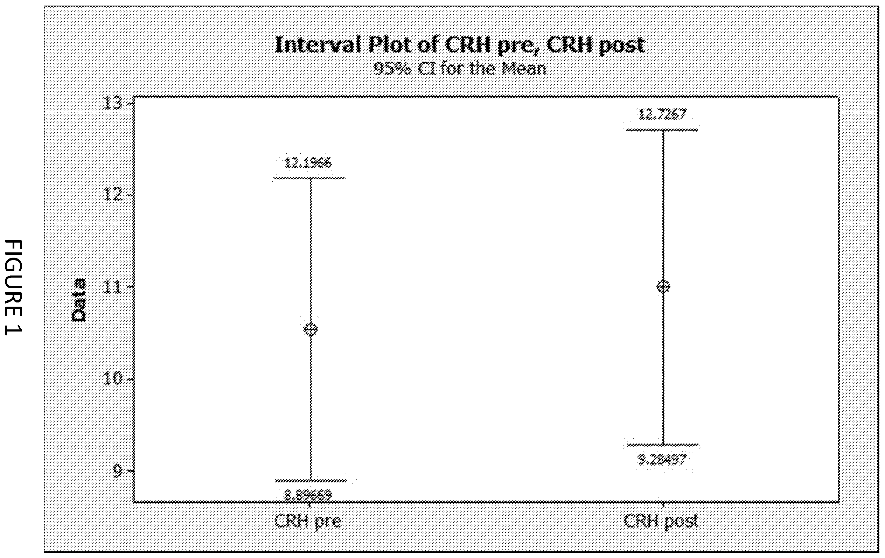

The mean results for the pre- and post-administration CRH data are shown in Figure 1. A paired T-test (data not shown) established (P=0.004) that the mean CRH concentration pre-administration is significantly less than the mean CRH concentration post-administration.

A test on a further ("control") group of patients established (data not shown) that there is no significant increase in CRH concentration following administration of a placebo.

### EXAMPLE 2

### Administration of the CRH formulation leads to a significant improvement in patients with multiple sclerosis

Following administration of the CRH formulation, the patient group and the control group were tested using the 9 Hole Peg Test ("9-HPT") and the Multiple Sclerosis Functional Composite (MSFC) test. These tests were carried out both prior to administration and four hours after administration of the CRH formulation or the placebo, as appropriate.

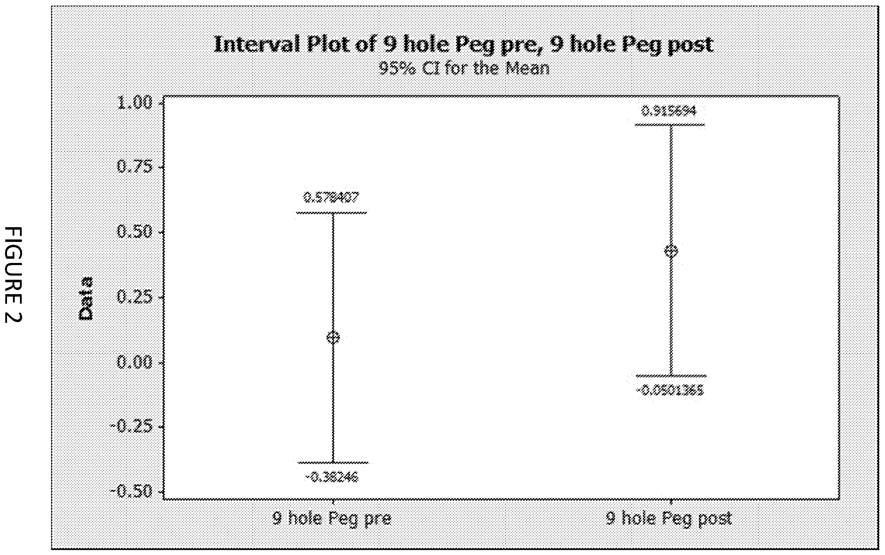

The mean results of the 9-HPT for the patient group administered the CRH formulation are shown in Figure 2. It was established (P=0.48, data not shown) that the data results follow a normal distribution. A paired T-test (data not shown) established (P=0.013) that there is a significant improvement in test results for the 9-HPT following administration of the CRH formulation.

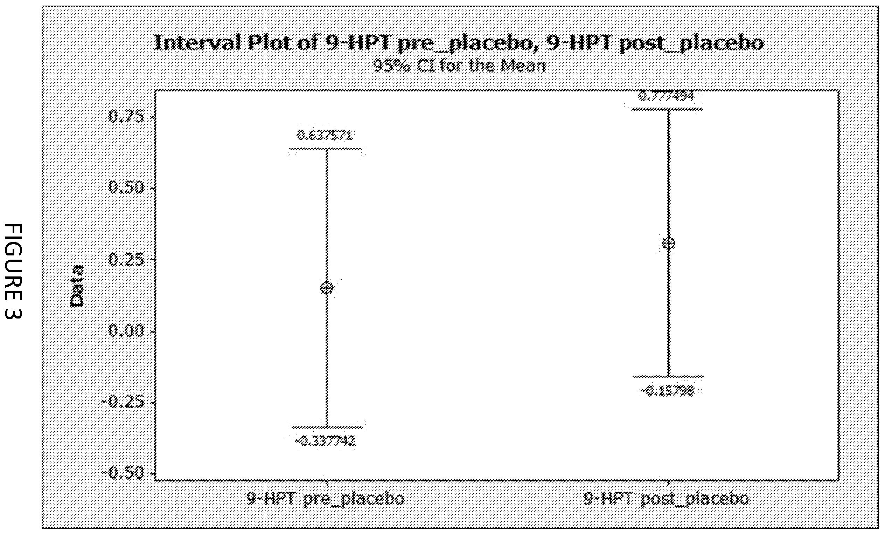

The mean results of the 9-HPT for the control group are shown in Figure 3. A paired T-test (data not shown) established (P=0.09) that there is no significant difference in test results for the 9-HPT following administration of the placebo.

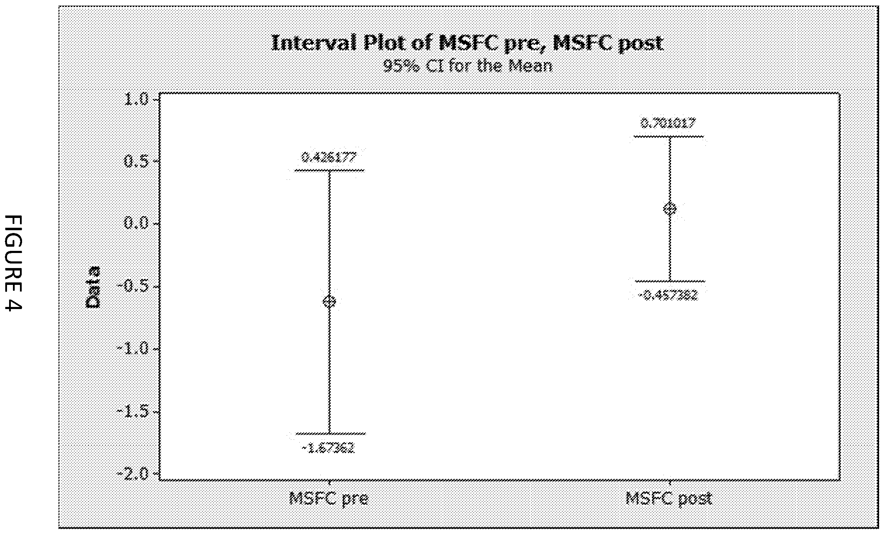

The mean results of the MSFC test for the patient group administered the CRH formulation are shown in Figure 4. A paired T-test (data not shown) established (P=0.045) that there is a significant improvement in test results for the MSFC test following administration of the CRH formulation.

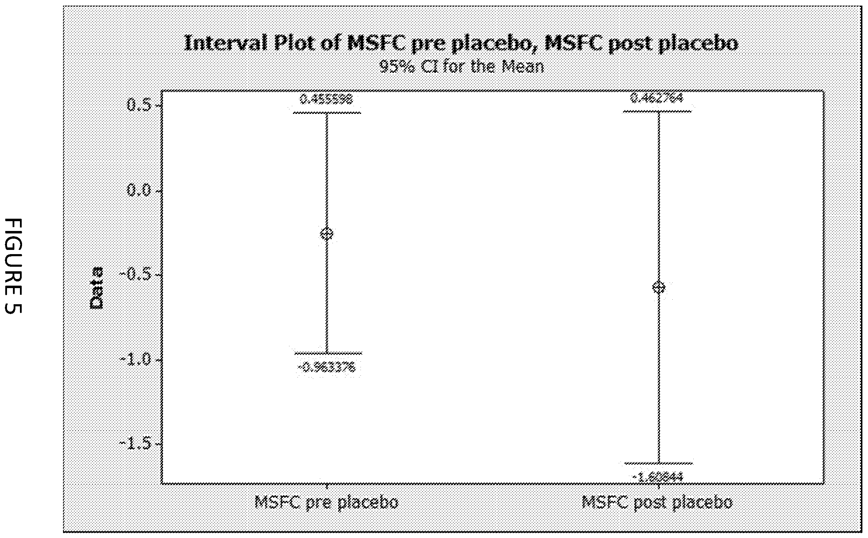

The mean results of the MSFC test for the control group are shown in Figure 5. A paired T-test (data not shown) established (P=0.461) that there is no significant difference in test results for the MSFC test following administration of the placebo.

### EXAMPLE 3

### Administration of the CRH formulation leads to a significant improvement in patients with systemic sclerosis

A further group of subjects suffering from systemic sclerosis was divided into a patient group to be administered the CRH formulation and a control group to be administered a placebo.

An MRSS assessment was carried out on both groups prior to administration. A further MRSS assessment was carried out on both groups 4 hours after administration.

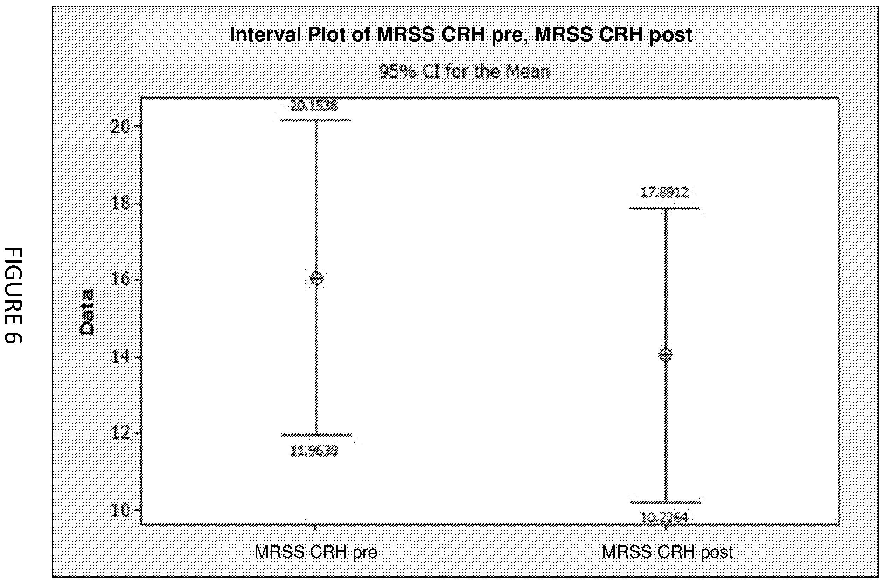

The mean results of the MRSS assessment for the patient group administered the CRH formulation are shown in Figure 6. It was established (data not shown) that the results follow a normal distribution (P=0.754). A paired T-test (data not shown) established (P=0.034) that there is a significant improvement in test results for the MRSS assessment following administration of the CRH formulation.

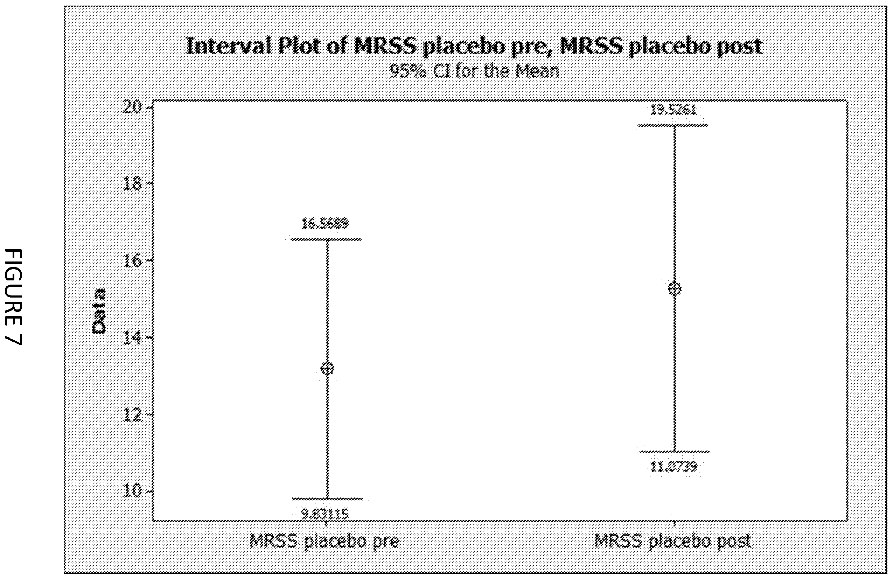

The mean results of the MRSS assessment for the control group are shown in Figure 7. A paired T-test (data not shown) established (P=0.837) that there is no significant improvement in test results for the MRSS assessment following administration of the placebo.

### EXAMPLE 4

### Increase in CRH following administration of the CRH formulation correlates with an improvement in patients

The data provided above show that there is an increase in CRH and that there is an improvement in patient test results following administration of the CRH formulation. It was investigated whether the two results are significantly correlated.

The relative increase in CRH concentration pre-and post-administration of the CRH formulation was plotted against the relative improvement in MSFC scores pre- and post-administration of the CRH formulation. An Analysis of Variance (ANOVA) test established (data not shown) that there is a significant (P=0.005) relationship between the increase in CRH concentration and the improvement in MSFC.

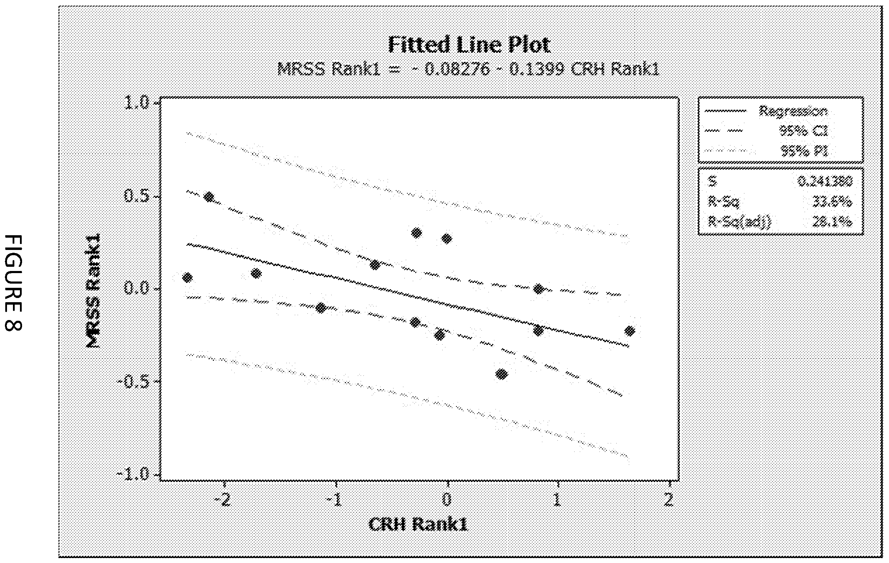

The relative increase in CRH concentration pre-and post-administration of the CRH formulation was plotted against the relative improvement in MRSS scores pre- and post-administration of the CRH formulation. The results are shown in Figure 8.

An Analysis of Variance (ANOVA) test established (data not shown) that there is a significant (P=0.03) relationship between the increase in CRH concentration and the improvement in MRSS.

### EXAMPLE 5

### Administration of the CRH formulation leads to an increase in concentration of other biomarkers

The above patient groups were also tested for their concentration of IL-17 and PIINP both pre- and post-administration of the CRH formulation. The relative change in concentration of these biomarkers was compared with the relative improvement in the MSFC test (for IL-17) and MRSS assessment (for PIIINP).

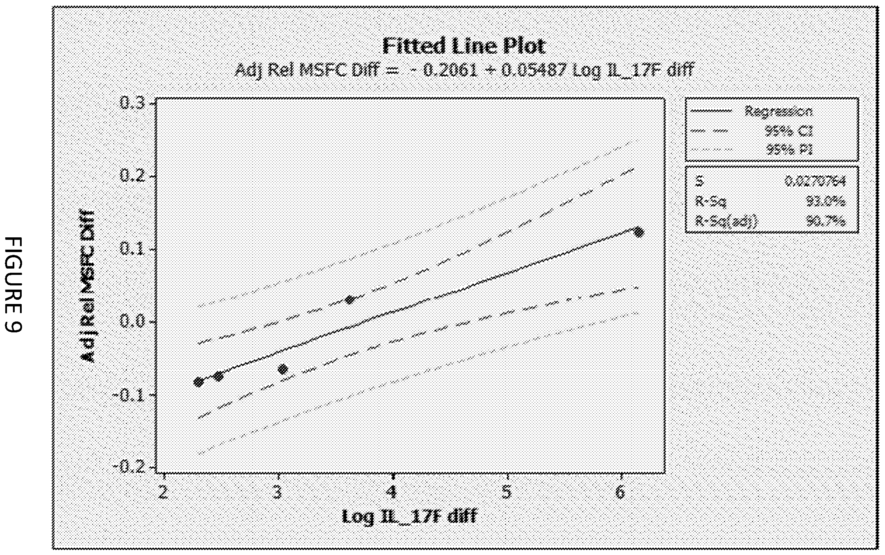

The relative increase in IL-17 concentration pre-and post-administration of the CRH formulation was plotted against the relative improvement in MSFC scores pre- and post-administration of the CRH formulation. The results are shown in Figure 9.

An Analysis of Variance (ANOVA) test established (data not shown) that there is a significant (P=0.008) relationship between the increase in IL-17 concentration and the improvement in MSFC.

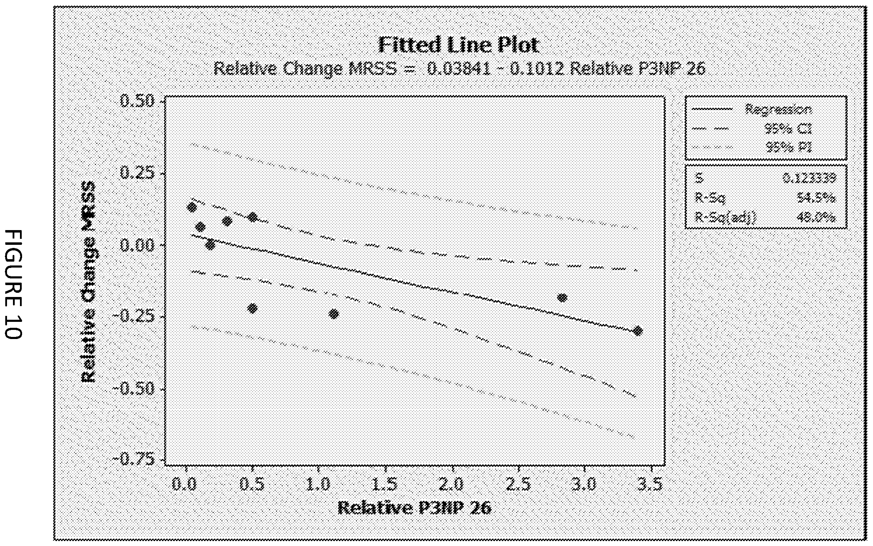

The relative increase in PIINP concentration pre-and post-administration of the CRH formulation was plotted against the relative improvement in MRSS scores pre- and post-administration of the CRH formulation. The results are shown in Figure 10.

An Analysis of Variance (ANOVA) test established (data not shown) that there is a significant (P=0.023) relationship between the increase in PIIINP concentration and the improvement in MRSS.

## Claims

1. A method for determining whether a patient is likely to benefit from treatment with a therapeutic formulation, the method comprising the steps of:
(a) determining the concentration of corticotropin releasing hormone (CRH) in a sample from a patient prior to administration of the therapeutic formulation;
(b) determining the concentration of CRH in a sample from a patient subsequent to administration of the therapeutic formulation; and
(c) comparing the concentration of CRH pre-administration with the concentration of CRH subsequent to administration;
wherein an increase in patient CRH concentration subsequent to administration indicates that the patient is likely to benefit from treatment with the therapeutic formulation and wherein no increase or a decrease in patient CRH concentration subsequent to administration indicates that the patient is unlikely to benefit from treatment with the therapeutic formulation;
wherein the therapeutic formulation comprises CRH.

2. The method according to claim 1, wherein step (b) is carried out within four hours of administration of the therapeutic formulation.

3. The method according to claim 1 or 2, wherein the therapeutic formulation is derived from an ungulate.

4. The method according to any one of the preceding claims, wherein the therapeutic formulation is derived from a goat.

5. The method according to any one of the preceding claims, wherein the therapeutic formulation is derived from a hyperimmune serum.

6. The method according to any one of the preceding claims, further comprising determining the concentration of PIIINP and/or IL-17 in a sample from a patient and comparing the concentration thereof pre-administration with the concentration thereof subsequent to administration, wherein an increase in said concentration subsequent to administration indicates that the patient is likely to benefit from treatment with the therapeutic formulation and wherein no increase or a decrease in said concentration subsequent to administration indicates that the patient is unlikely to benefit from treatment with the therapeutic formulation.

7. The method according to any one of the preceding claims, wherein the patient has multiple sclerosis.

8. The method according to claim 7 when dependent upon claim 6, wherein the method comprises measuring the concentration of CRH and IL-17 only.

9. The method according to any one of the preceding claims, wherein the patient has systemic sclerosis.

10. The method according to claim 9 when dependent upon claim 6, wherein the method comprises measuring the concentration of CRH and PIIINP only.

11. The method according to any one of the preceding claims, wherein the patient has one or more of the disorders selected from the following list: an inflammatory disorder such as rheumatoid arthritis; optic neuritis; motor neuron disease; autoimmune diseases; axonal or nerve damage; and cancers (including myelomas, melanomas and lymphomas); cardiovascular diseases; neural disorders, both demyelinating and non-demyelinating; cerebrovascular ischemic disease; Alzheimer's disease; Parkinson's disease; Huntingdon's chorea; mixed connective tissue diseases; scleroderma; anaphylaxis; septic shock; carditis and endocarditis; wound healing; contact dermatitis; occupational lung diseases; glomerulonephritis; transplant rejection; temporal arteritis; vasculitic diseases; hepatitis (in particular hepatitis C); burns; multiple system atrophy; epilepsy; muscular dystrophy; schizophrenia; bipolar disorder; depression; channelopathies; myasthenia gravis; pain due to malignant neoplasia; chronic fatigue syndrome; fibromyositis; irritable bowel syndrome; work related upper limb disorder; cluster headache; migraine; chronic daily headache; infections of the nervous system; nerve entrapment and focal injury; traumatic spinal cord injury; brachial plexopathy (idiopathic and traumatic, brachial neuritis, parsonage turner syndrome, neuralgic amyotrophy); radiculopathy; channelopathies; tic douloureux; lupus; psoriasis; eczema; thyroiditis; polymysotis; hereditary motor and sensor neuropathy of all types; Charcot-Marie-Tooth disease (CMT) types CMT1A, CMT1B, CMT2, CMT3 (Dejerine Sottas disease), CMT4 (Types A, B, C and D), X-linked Charcot-Marie-Tooth disease (CMTX); Hereditary Neuropathy with liability to pressure palsies (HNPP), also called Tomaculous neuropathy; Hereditary Motor and Sensory Neuropathy with Deafness - Lom (HMSNL); Proximal Hereditary Motor and Sensory Neuropathy/Neuronopathy (HMSNP); Hereditary Neuralgic Amyotrophy; Hereditary Sensory and Autonomic Neuropathies (HSAN1, HSAN2, HSAN3 (also called Riley-Day syndrome or familial dysautonomia), HSAN4, HSAN5); Familial Amyloid polyneuropathies (Type I, Type II, Type III, Type IV); Metachromatic Leukodystrophy; Krabbe's Disease; Fabry's Disease; Adrenoleukodystrophy; Refsum's disease (HMSN IV); Tangier Disease; Friedreich's ataxia; Spinal cerebellar ataxia (SCA) all types - SCA1, SCA2, SCA3, SCA4, SCA5, SCA6, SCA7, SCA8, SCA10, SCA11, SCA12, SCA13, SCA14, SCA16; Spinocerebellar Ataxia; Cockayne's syndrome; Giant axonal neuropathy; chronic inflammatory demyelinating polyneuropathy (CIDP); and Guillain-Barre syndrome.

12. The method according to any one of the preceding claims, wherein the patient is a non-ungulate and the therapeutic formulation contains an ungulate CRH.

13. The method according to any one of the preceding claims, wherein the patient is a human and the therapeutic formulation contains a non-human CRH.

14. The method according to claim 13, wherein the non-human CRH is an ungulate CRH.

## Patentansprüche

1. Verfahren zur Bestimmung, ob ein Patient wahrscheinlich von einer Behandlung mit einer therapeutischen Formulierung profitieren wird, wobei das Verfahren die folgenden Schritte umfasst:
(a) Bestimmen der Konzentration von Corticotropinfreisetzendem Hormon (CRH) in einer Probe von einem Patienten vor einer Verabreichung der therapeutischen Formulierung;
(b) Bestimmen der Konzentration von CRH in einer Probe von einem Patienten nach der Verabreichung der therapeutischen Formulierung und
(c) Vergleichen der Konzentration von CRH vor der Verabreichung mit der Konzentration von CRH nach der Verabreichung;
wobei ein Anstieg der Patienten-CRH-Konzentration nach der Verabreichung angibt, dass der Patient wahrscheinlich von einer Behandlung mit der therapeutischen Formulierung profitieren wird, und wobei kein Anstieg oder ein Rückgang der Patienten-CRH-Konzentration nach der Verabreichung angibt, dass der Patient wahrscheinlich nicht von einer Behandlung mit der therapeutischen Formulierung profitieren wird;
wobei die therapeutische Formulierung CRH umfasst.

2. Verfahren nach Anspruch 1, wobei Schritt (b) innerhalb von vier Stunden der Verabreichung der therapeutischen Formulierung durchgeführt wird.

3. Verfahren nach Anspruch 1 oder 2, wobei die therapeutische Formulierung von einem Huftier abgeleitet ist.

4. Verfahren nach einem der vorhergehenden Ansprüche, wobei die therapeutische Formulierung von einer Ziege abgeleitet ist.

5. Verfahren nach einem der vorhergehenden Ansprüche, wobei die therapeutische Formulierung von einem Hyperimmunserum abgeleitet ist.

6. Verfahren nach einem der vorhergehenden Ansprüche, weiterhin umfassend ein Bestimmen der Konzentration von PIIINP und/oder IL-17 in einer Probe von einem Patienten und ein Vergleichen der Konzentration davon vor einer Verabreichung mit der Konzentration davon nach der Verabreichung, wobei ein Anstieg der genannten Konzentration nach der Verabreichung angibt, dass der Patient wahrscheinlich von einer Behandlung mit der therapeutischen Formulierung profitieren wird, und wobei kein Anstieg oder ein Rückgang der genannten Konzentration nach der Verabreichung angibt, dass der Patient wahrscheinlich nicht von einer Behandlung mit der therapeutischen Formulierung profitieren wird.

7. Verfahren nach einem der vorhergehenden Ansprüche, wobei der Patient an multipler Sklerose leidet.

8. Verfahren nach Anspruch 7 bei Abhängigkeit von Anspruch 6, wobei das Verfahren ein Messen der Konzentration von nur CRH und IL-17 umfasst.

9. Verfahren nach einem der vorhergehenden Ansprüche, wobei der Patient an systemischer Sklerose leidet.

10. Verfahren nach Anspruch 9 bei Abhängigkeit von Anspruch 6, wobei das Verfahren ein Messen der Konzentration von nur CRH und PIIINP umfasst.

11. Verfahren nach einem der vorhergehenden Ansprüche, wober der Patient an einer oder mehreren der Erkrankungen leidet, die aus der folgenden Liste ausgewählt sind: einer Entzündungserkrankung, wie rheumatoide Arthritis; Optikusneuritis; Motoneuron-Krankheit; Autoimmunkrankheiten; Axon- oder Nervenschädigung und Krebserkrankungen (einschließlich von Myelomen, Melanomen und Lymphomen); Herz-Kreislauf-Krankheiten; Nervenerkrankungen, sowohl demyelinisierend als auch nichtdemyelinisierend; zerebrovaskulärer ischämischer Krankheit; Alzheimer-Krankheit; Parkinson-Krankheit; Morbus Huntington; Sharp-Syndrom; Skleroderm; Anaphylaxie; septischer Schock; Karditis und Endokarditis; Wundheilung; Kontaktdermatitis; beruflich bedingten Lungenkrankheiten; Glomerulonephritis; Transplantatabstoßung; Riesenzellarteriitis; Vaskulitiskrankheiten; Hepatitis (insbesondere Hepatitis C); Verbrennungen; multipler Systematrophie; Epilepsie; Muskeldystrophie; Schizophrenie; bipolarer Erkrankung; Depression; Channelopathien; Erb-Goldflam-Syndrom; Schmerzen aufgrund von maligner Neoplasie; chronischem Ermüdungssyndrom; Fibromyositis; Reizkolon; arbeitsbedingten Erkrankungen der oberen Gliedmaßen; Horton-Syndrom; Migräne; chronischem täglichem Kopfschmerz; Infektionen des Nervensystems; Nerveneinklemmung und fokalen Verletzungen; traumatischer Rückenmarksverletzung; brachialer Plexopathie (idiopathisch und traumatisch, brachiale Neuritis, Parsonage-Turner-Syndrom, neuralgische Amyotrophie); Radikulopathie; Channelopathien; Trigeminusneuralgie; Lupus; Psoriasis; Ekzem; Thyroiditis; Polymyositis; hereditärer motorischer und sensorischer Neuropathie aller Typen; Charcot-Marie-Tooth-Hoffmann-Krankheit (CMT) Typen CMT1A, CMT1B, CMT2, CMT3 (Dejerine-Sottas-Krankheit), CMT4 (Typen A, B, C und D), X-gekoppelte Charcot-Marie-Tooth-Hoffmann-Krankheit (CMTX); hereditärer Neuropathie mit Neigung zu Drucklähmung (HNPP), auch tomakulöse Neuropathie genannt; hereditärer motorischer und sensorischer Neuropathie mit Taubheit - Lom (HMSNL); proximaler hereditärer motorischer und sensorischer Neuropathie/Neuronopathie (HMSNP); hereditärer neuralgischer Amyotrophie; hereditären sensorischen und autonomen Neuropathien (HSAN1, HSAN2, HSAN3 (auch Riley-Day-Syndrom oder familiäre Dysautonomie genannt), HSAN4, HSAN5); familiären Amyloidpolyneuropathien (Typ I, Typ II, Typ III, Typ IV); metachromatischer Leukodystrophie; Krabbe-Syndrom; Fabry-Syndrom; Adrenoleukodystrophie; Refsum-Syndrom (HMSN IV); Tangier-Krankheit; Friedreich-Ataxie; spinaler zerebellärer Ataxi (SCA), alle Typen - SCA1, SCA2, SCA3, SCA4, SCA5, SCA6, SCA7, SCA8, SCA10, SCA11, SCA12, SCA13, SCA14, SCA16; spinozerebellärer Ataxie; Cockayne-Syndrom; Riesenaxonneuropathie; chronisch inflammatorischer demyelinisierender Polyneuropathie (CIDP) und Guillain-Barre-Syndrom.

12. Verfahren nach einem der vorhergehenden Ansprüche, wobei der Patient kein Huftier ist und die therapeutische Formulierung ein Huftier-CRH enthält.

13. Verfahren nach einem der vorhergehenden Ansprüche, wobei der Patient ein Mensch ist und die therapeutische Formulierung ein nichtmenschliches CRH enthält.

14. Verfahren nach Anspruch 13, wobei das nichtmenschliche CRH ein Huftier-CRH ist.

## Revendications

1. Procédé destiné à déterminer si un patient est susceptible de tirer bénéfice d'un traitement avec une formulation thérapeutique, le procédé comprenant les étapes de :
(a) détermination de la concentration d'hormone de libération de la corticotropine (CRH) dans un échantillon d'un patient avant l'administration de la formulation thérapeutique ;
(b) détermination de la concentration de CRH dans un échantillon d'un patient après l'administration de la formulation thérapeutique ; et
(c) comparaison de la concentration de CRH avant l'administration avec la concentration de CRH après l'administration ;
dans lequel une augmentation de la concentration de CRH du patient après l'administration indique que le patient est susceptible de tirer bénéfice du traitement avec la formulation thérapeutique et dans lequel l'absence d'augmentation ou une diminution de la concentration de CRH du patient après l'administration indique que le patient n'est pas susceptible de tirer bénéfice du traitement avec la formulation thérapeutique ;
dans lequel la formulation thérapeutique comprend de la CRH.

2. Procédé selon la revendication 1, dans lequel l'étape (b) est réalisée dans les quatre heures qui suivent l'administration de la formulation thérapeutique.

3. Procédé selon la revendication 1 ou 2, dans lequel la formulation thérapeutique est dérivée d'un ongulé.

4. Procédé selon l'une quelconque des revendications précédentes, dans lequel la formulation thérapeutique est dérivée d'une chèvre.

5. Procédé selon l'une quelconque des revendications précédentes, dans lequel la formulation thérapeutique est dérivée d'un sérum hyperimmun.

6. Procédé selon l'une quelconque des revendications précédentes, comprenant en outre la détermination de la concentration de P-111-NP et/ou d'IL-17 dans un échantillon d'un patient et la comparaison de la concentration de ceux-ci avant l'administration avec la concentration de ceux-ci après l'administration, dans lequel une augmentation de ladite concentration après l'administration indique que le patient est susceptible de tirer bénéfice du traitement avec la formulation thérapeutique et dans lequel l'absence d'augmentation ou la diminution de ladite concentration après l'administration indique que le patient n'est pas susceptible de tirer bénéfice du traitement avec la formulation thérapeutique.

7. Procédé selon l'une quelconque des revendications précédentes, dans lequel le patient est atteint de sclérose en plaques.

8. Procédé selon la revendication 7 quand elle est dépendante de la revendication 6, dans lequel le procédé comprend la mesure de la concentration de CRH et d'IL-17 uniquement.

9. Procédé selon l'une quelconque des revendications précédentes, dans lequel le patient est atteint de sclérose systémique.

10. Procédé selon la revendication 9 quand elle est dépendante de la revendication 6, dans lequel le procédé comprend la mesure de la concentration de CRH et de P-111-NP uniquement.

11. Procédé selon l'une quelconque des revendications précédentes, dans lequel le patient est atteint d'un ou de plusieurs des troubles choisis parmi la liste suivante : un trouble inflammatoire tel que la polyarthrite rhumatoïde ; la névrite optique ; la maladie du neurone moteur ; les maladies auto-immunes ; la lésion axionale ou nerveuse ; et les cancers (incluant les myélomes, les mélanomes et les lymphomes) ; les maladies cardiovasculaires ; les troubles neuraux, tant démyélinisants que non-démyélinisants ; la maladie vasculaire cérébrale ischémique ; la maladie d'Alzheimer ; la maladie de Parkinson ; la chorée de Huntington ; les maladies du tissu conjonctif mixtes ; la sclérodermie ; l'anaphylaxie ; le choc septique ; la cardite et l'endocardite ; la cicatrisation ; la dermatite de contact ; les maladies pulmonaires professionnelles ; la glomérulonéphrite ; le rejet de greffe ; l'artérite temporale ; les maladies vasculitiques ; l'hépatite (en particulier l'hépatite C) ; les brûlures ; l'atrophie multisystématisée ; l'épilepsie ; la dystrophie musculaire ; la schizophrénie ; le trouble bipolaire ; la dépression ; les canalopathies ; la myasthénie grave ; la douleur due à la néoplasie maligne ; le syndrome de fatigue chronique ; la fibromyosite ; le syndrome du côlon irritable ; le trouble des membres supérieurs lié au travail ; la céphalée vasculaire de Horton ; la migraine ; le mal de tête quotidien chronique ; les infections du système nerveux ; la compression de nerf et la lésion focale ; la lésion de la moelle épinière d'origine traumatique ; la plexopathie brachiale (idiopathique et traumatique, la névrite brachiale, le syndrome de Parsonage-Turner, l'amyotrophie névralgique) ; la radiculopathie ; les canalopathies ; le tic douloureux ; le lupus ; le psoriasis ; l'eczéma ; la thyroïdite ; la polymyosite ; la neuropathie motrice et sensitive héréditaire de tous les types ; la maladie de Charcot-Marie-Tooth (CMT) des types CMT1A, CMT1B, CMT2, CMT3 (maladie de Dejerine-Sottas), CMT4 (Types A, B, C et D), la maladie de Charcot-Marie-Tooth liée à l'X (CMTX) ; la neuropathie héréditaire avec paralysies à la pression (HNPP), aussi désignée par la neuropathie tomaculaire ; la neuropathie motrice et sensitive héréditaire avec surdité de type Lom (HMSNL) ; la neuropathie/neuronopathie motrice et sensitive héréditaire proximale (HMSNP) ; l'amyotrophie névralgique héréditaire ; les neuropathies sensitives et autonomes héréditaires (HSAN1, HSAN2, HSAN3 (aussi désignées par le syndrome de Riley-Day ou la dysautonomie familiale), HSAN4, HSAN5) ; les polyneuropathies amyloïdes familiales (Type I, Type II, Type III, Type IV) ; la leucodystrophie métachromatique ; la maladie de Krabbe ; la maladie de Fabry ; l'adrénoleucodystrophie ; la maladie de Refsum (HMSN IV) ; la maladie de Tangier ; l'ataxie de Friedreich ; l'ataxie cérébelleuse spinale (SCA) de tous les types - SCA 1, SCA2, SCA3, SCA4, SCA5, SCA6, SCA7, SCA8, SCA10, SCA11, SCA12, SCA13, SCA14, SCA16 ; l'ataxie spinocérébelleuse ; le syndrome de Cockayne ; la neuropathie à axones géants ; la polyneuropathie démyélinisante inflammatoire chronique (CIDP) ; et le syndrome de Guillain-Barré.

12. Procédé selon l'une quelconque des revendications précédentes, dans lequel le patient n'est pas un ongulé et la formulation thérapeutique contient une CRH d'ongulé.

13. Procédé selon l'une quelconque des revendications précédentes, dans lequel le patient est un humain et la formulation thérapeutique contient une CRH non humaine.

14. Procédé selon la revendication 13, dans lequel la CRH non humaine est une CRH d'ongulé.
